# EUROPEAN PATENT SPECIFICATION

(11) **EP 4 333 745 B1**
(45) Date of publication and mention of the grant of the patent: **09.12.2026**
(21) Application number: 22799249.2
(22) Date of filing: 15.02.2022
(51) Int. Cl.: A61B 17/34, A61M 1/00, A61M 13/00

(54) **ACTIVE SURGICAL ACCESS PORT FILTRATION FITTINGS**
FILTRATIONSARMATUREN FÜR AKTIVEN CHIRURGISCHEN ZUGANG
RACCORDS DE FILTRATION À ORIFICE D'ACCÈS CHIRURGICAL ACTIF

(30) Priority: 05.05.2021 US 202117308255
(43) Date of publication of application: 13.03.2024
(73) Proprietor: CONMED Corporation, Largo, FL 33773 (US)
(72) Inventor: LATHERS, Steven, Littleton, CO 80128 (US); KRISHNAMOORTHY, Mahesh, Parker, CO 80134 (US)
(74) Representative: Hoefer & Partner Patentanwälte mbB
(86) International application number: PCT/US2022/016467
(87) International publication number: WO 2022/235317

(56) References cited:
- EP-B1- 3 360 494
- WO-A1-2020/036497
- US-A1- 2009 137 943
- US-A1- 2018 228 510
- US-A1- 2018 256 830
- US-A1- 2020 187 984
- US-A1- 2020 305 928
- US-B1- 10 492 827
- US-B2- 6 685 665

## Description

### BACKGROUND

### 1. Field

The present disclosure relates to active filtration, and more particularly to active filtration for surgical access ports such as used with insufflation systems.

### 2. Description of Related Art

Some surgical access ports are designed as open, valve-less trocars with gas pressure barriers to prevent the loss of pneumoperitoneum. AirSeal^{®} Access Ports as part of AirSeal^{®} iFS insufflation management systems available from ConMed Corporation of Utica, New York can be introduced to a patient's surgical site during an insufflation procedure. Sometimes surgeons use AirSeal^{®} iFS insufflation management systems in procedures where the AirSeal^{®} Access Port is not used for access to the surgical site, e.g. when no surgical instruments are inserted through the trocar of the AirSeal^{®} Access Port during in the procedure. In this scenario, the AirSeal^{®} iFS insufflation management system provides the insufflation for the procedure. The AirSeal^{®} System provides for stable pneumoperitoneum, and can also provide for smoke evacuation.

EP 3 360 494 describes a seal assembly for use with an access apparatus includes an integral filter and evacuation port for filtering of fluids, e.g., smoke, from an operating site and removing contaminants and/or odor from the fluids for release of the filtered fluids into the ambient atmosphere. The seal assembly defines its own flow path independent of the insufflation mechanism of the cannula assembly, and is capable of filtering fluids even in the presence of a surgical object positioned within the cannula assembly.

Regardless of the type of surgical access port used, due to the open nature of some access port designs, when there is no instrument passing through an access port there is an opportunity for particles to be emitted from inside a patient, through the access port opening, and into the operating room air. This can allow for unwanted or harmful particles to move from the intraabdominal cavity or other surgical site to the operating room where surgical staff/employees are present. This could include gasses or even pathogens that are harmful to the surgical staff/employees, who would have to rely on their PPE (personal protective equipment) for protection.

The conventional techniques have been considered satisfactory for their intended purpose. However, there is an ever present need for improved systems and methods for preventing unwanted or harmful particles from entering the operating room air through surgical access ports. This disclosure provides a solution for this need.

### SUMMARY

The invention is defined by the independent claim 1. Further embodiments are defined by the dependent claims. No methods are claimed. A cap for a trocar assembly includes a housing configured to be removably attached to a proximal portion of a trocar. The housing defines an access port opening therethrough configured to be aligned for passage of a surgical instrument through the access port opening and into a main access port of the trocar. The housing defines a siphon opening for siphoning particles from the proximal portion of the trocar for filtration.

A plenum is defined in the cap radially outward from the access port opening with a plenum wall separating between the plenum and the access port opening. The siphon opening is in fluid communication with the plenum to siphon particles out of the plenum. At least one port is defined through the plenum wall for fluid communication from the access port opening into the plenum. The at least one port, the plenum and the siphon opening are arranged in a common plane that is perpendicular to the longitudinal axis of the access port opening. A tube set can be included having a siphon tube connected to the siphon opening.

The siphon tube can connect for fluid communication between the housing and an external filter unit. The external filter unit can house a filter medium spanning a flow passage through the external filter unit for filtration of flow through the flow passage. The external filter unit can include an outlet tube for connecting to an insufflator for active filtration.

It is also contemplated that the tube set can include an insufflation line and a smoke evacuation line, each connecting between a cartridge and a multiport connector. The cartridge can be configured to connect the tube set to an insufflator. The multiport connector can be configured to connect the insufflation line and the smoke evacuation line to a trocar for insufflation and smoke evacuation. The siphon tube can connect to the smoke evacuation line for active filtration of particles from the trocar by the filter medium. The cartridge can include an ultra-low particulate air (ULPA) filter medium in fluid communication with the siphon line for filtration of particles from the trocar.

The housing can define an inlet opening in fluid communication with the access port opening, wherein the inlet opening is configured to engage the trocar. A seal can extend circumferentially around the inlet opening. The seal can be configured to engage the proximal portion of the trocar to drive all flow into and out of a main lumen of the trocar through the inlet opening. A seal seat can be defined about the inlet opening of the housing. The seal can be seated in the seal seat.

A distal end of the housing can include at least one inward extending latch member configured to engage a respective rim or detent of the trocar to maintain engagement of the housing to the trocar. It is also contemplated that the distal end of the housing can include a plurality of circumferentially spaced apart, inward extending latch members configured to engage a respective rim or detent of the trocar to maintain engagement of the housing to the trocar.

A trocar assembly includes a trocar. The trocar includes an elongated tubular member extending between a distal end configured to be inserted into a surgical site and a proximal portion including a trocar housing configured for introduction of surgical instruments into the tubular member. The trocar housing includes at least one latch receptacle. A cap as described above is included, wherein the housing of the cap is attached to the proximal portion of the trocar. A kit includes a trocar as described above, and a cap as described above, wherein the trocar and cap are attached or separate from one another in the kit.

A method includes regulating insufflation of a surgical site with a trocar introduced into the surgical site. The method includes siphoning fluid that vents out of the surgical site through the trocar into a tube set, thereby diverting the fluid from reaching a space external of the surgical site.

The method can include capturing liquid droplets, solid particulate, and/or gas from the fluid in a filter medium in a flow path of the fluid. The filter medium can be in a fluid circuit connecting between the trocar and an insufflator regulating insufflation with the trocar and/or conducting smoke evacuation through the trocar. The filter medium can be within an insufflator regulating insufflation with the trocar and/or conducting smoke evacuation through the trocar. The method can include evacuating smoke from the surgical site through the trocar and/or regulating stable pneumoperitoneum using the trocar.

The trocar can be a first access port, and the method can include accessing the surgical site through second access port. Accessing the surgical site through the second access port can include accessing the surgical site without accessing the surgical site through the first access port.

These and other features of the systems and methods of the subject disclosure will become more readily apparent to those skilled in the art from the following detailed description of the preferred embodiments taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, preferred embodiments thereof will be described in detail herein below with reference to certain figures, wherein:
Fig. 1 is a schematic perspective view of an embodiment of a trocar assembly constructed in accordance with the present disclosure, showing the trocar assembly in situ during a procedure on a patient using insufflation;
Fig. 2 is a perspective view of the trocar assembly of Fig. 1, showing the passive filtration cap in place on the trocar;
Fig. 3 is an exploded perspective view of the trocar assembly of Fig. 2, showing the cap removed from the trocar;
Fig. 4 is a cross-sectional side elevation view of the cap of Fig. 3, showing the access port opening and the siphon opening communicating through a plenum;
Fig. 5 is a cross-sectional perspective view of the cap of Fig. 3, showing the plenum wall separating between the access port opening and the plenum;
Fig. 6 is a schematic perspective view of another embodiment of a trocar assembly constructed in accordance with the present disclosure, showing the trocar assembly in situ during a procedure on a patient using insufflation, with an external filter for capture of particulate from the siphon opening; and
Fig. 7 is a is a perspective view of an embodiment of a kit including parts for the trocar assembly of Fig. 1.

### DETAILED DESCRIPTION OF THE PREFERRED EMBODIMENTS

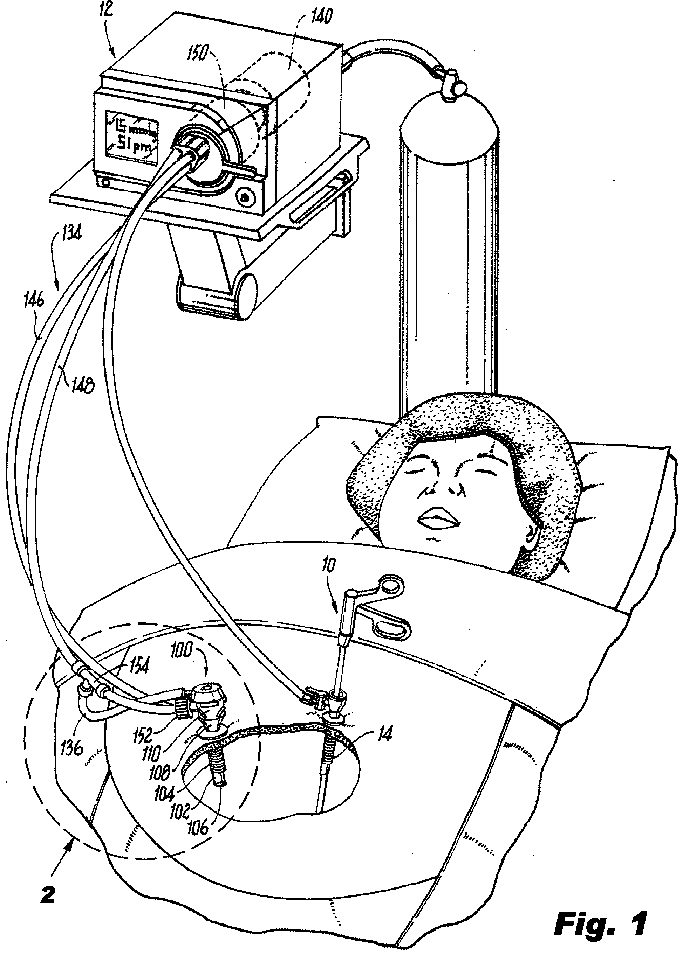

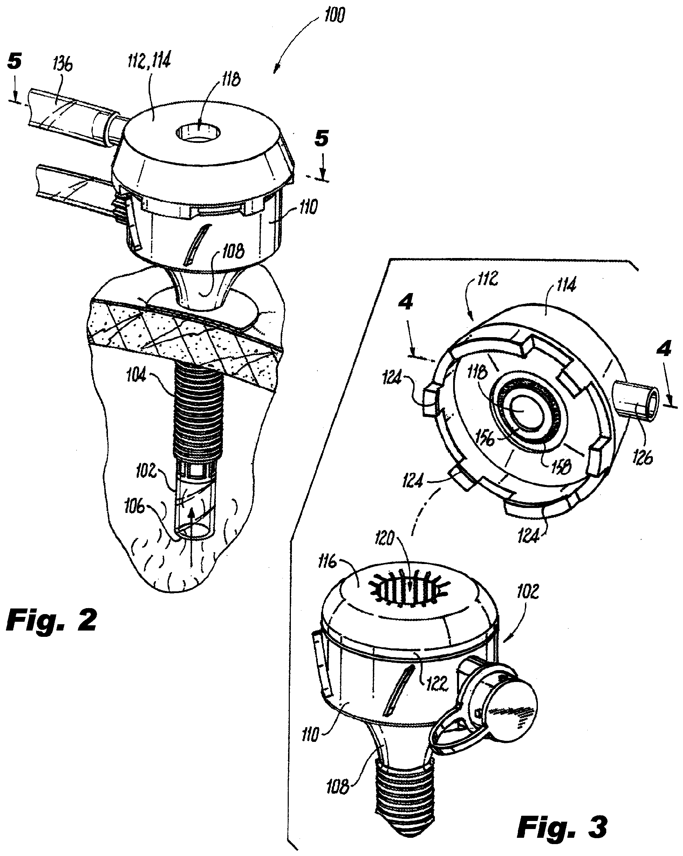

Reference will now be made to the drawings wherein like reference numerals identify similar structural features or aspects of the subject disclosure. For purposes of explanation and illustration, and not limitation, a partial view of an embodiment of a trocar assembly in accordance with the disclosure is shown in Fig. 1 and is designated generally by reference character 100. Other embodiments of systems in accordance with the disclosure, or aspects thereof, are provided in Figs. 2-7, as will be described. The systems and methods described herein can be used to provide active filtration to prevent particles from within a pneumoperitoneum from entering the operating room air without impeding the performance or effectiveness of the insufflation or stable pneumoperitoneum.

The trocar assembly 100 includes a trocar 102. The trocar includes an elongated tubular member 104 extending between a distal end 106 configured to be inserted into a surgical site and a proximal portion 108 including a trocar housing 110 configured for introduction of surgical instruments, e.g. surgical instrument 11, into the tubular member 104.

With reference now to Fig. 2, the assembly 100 includes a cap 112. The cap 112 includes a cap housing 114 configured to be removably attached to a proximal portion 108 of the trocar 102, e.g. to a proximal end 116 of the trocar housing 110. The housing 114 defines an access port opening 118 therethrough configured to be aligned for passage of a surgical instrument, e.g. surgical instrument 11 of Fig. 1, through the access port opening 118 and into a main access port 120 of the trocar 102, which is labeled in Fig. 3.

With continued reference to Fig. 3, the trocar housing 110 includes at least one latch receptacle 122 in the form of a rim or detent. A distal end of the housing 114 includes a plurality of circumferentially spaced apart inward extending latch members 124 configured to engage the latch receptacle 122 of the trocar 102 to maintain engagement of the cap 112 to the trocar 102. The housing 114 defines a siphon opening 126 for siphoning particles from the proximal portion 108 of the trocar 102 for filtration.

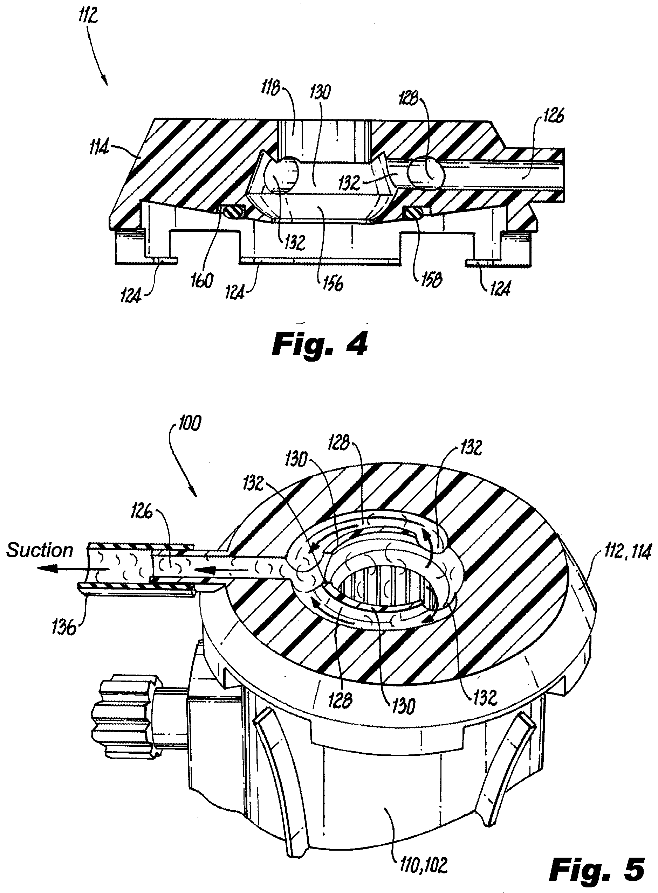

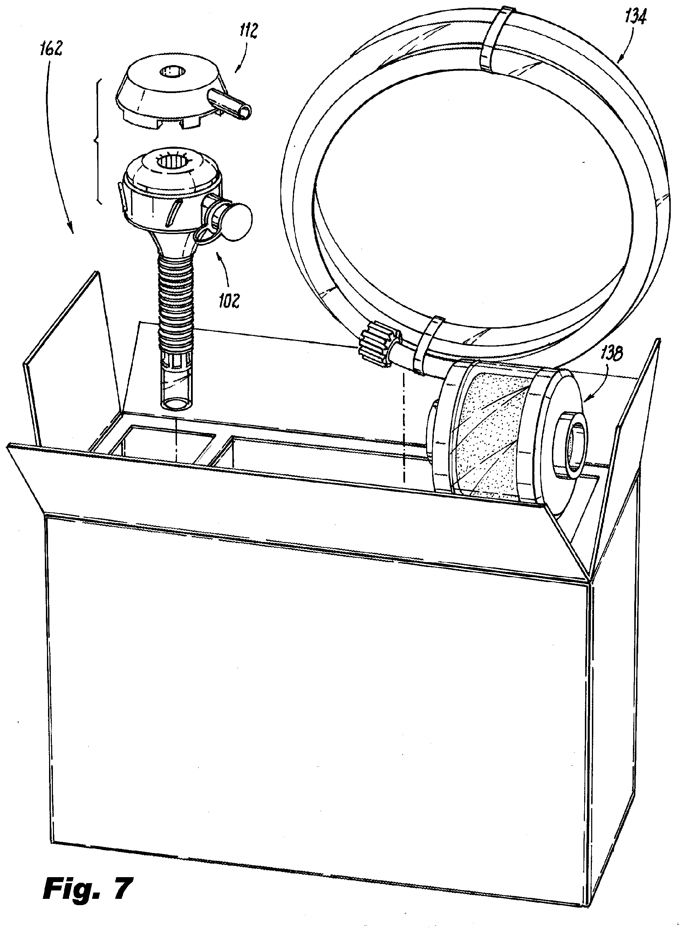

With reference now to Fig. 4, a plenum 128 is defined in the cap 112 radially outward from the access port opening 118 with a plenum wall 130 separating between the plenum 128 and the access port opening 118. The plenum wall is shown in cross-section in Fig. 5. The siphon opening 126 is in fluid communication with the plenum 128 to siphon particles out of the plenum 128, and ultimately from the access port opening 118. At least one port 132 is defined through the plenum wall 130 for fluid communication from the access port opening 118 into the plenum 128. In Figs. 4-5, there are three ports 132 through the plenum wall 130, however any suitable number of ports can be used without departing from the scope of this disclosure. A tube set 134 (e.g. as shown in Fig. 7) is included, having a siphon tube 136 configured to connect to the siphon opening 126 when in use.

With continued reference to Fig. 4, the cap housing 114 defines an inlet opening 156 in fluid communication with the access port opening 118, wherein the inlet opening 156 is configured to engage the trocar 102 of Figs. 1-3. A seal 158 extends circumferentially around the inlet opening 156. The seal 158 is configured to engage the proximal end 116 of the proximal portion 108 of the trocar 102 (as labeled in Fig. 3) to drive all flow into and out of a main lumen of the trocar 102 through the inlet opening 156. A seal seat 160 is defined about the inlet opening 156, and the seal 158 is seated in the seal seat 160. It is also contemplated that in addition to or in lieu of seal 158 and seal seat 160, a polymer adhesive can be applied around the inlet opening 156 to seal with the trocar 102.

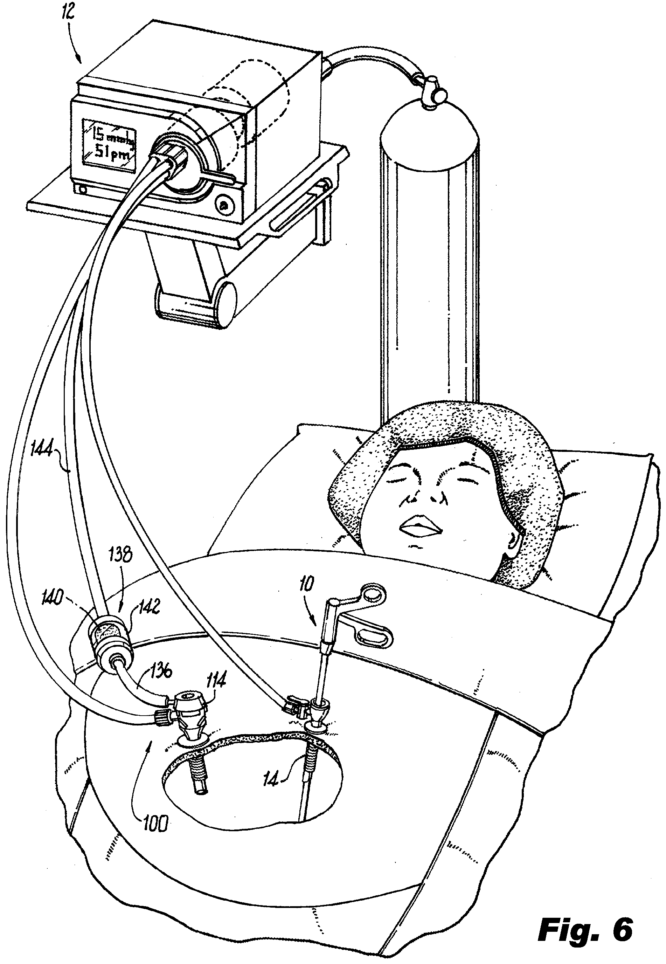

With reference to Fig. 6, the siphon tube 136 can connect for fluid communication between the cap housing 114 and an external filter unit 138. The external filter unit 138 houses a filter medium 140, e.g., an ultra-low particulate air (ULPA) filter medium, spanning a flow passage 142 through the external filter unit 138 for filtration of flow through the flow passage 142. The external filter unit 138 includes an outlet tube 144 for connecting to an insufflator 12 for active filtration of particles from the housing 114, e.g. to reduce or prevent those particles escaping into the operating room. The outlet tube 144 can be connected to any suitable source of suction, such as a smoke evacuation system in an insufflator 12, a central surgical vacuum system, or the like.

It is also contemplated that the external filter unit 138 need not be included, for example as shown in Fig. **1****,** where the tube set 134 includes a pair of insufflation lines 146 and a smoke evacuation line 148, each connecting between a cartridge 150 and a multiport connector 152. The cartridge 150 is configured to connect the tube set 134 to an insufflator 12. The multiport connector 152 is configured to connect the insufflation lines 146 and the smoke evacuation line 148 to the trocar housing 110 of the trocar 102 for insufflation and smoke evacuation. The siphon tube 136 connects to the smoke evacuation line 148, e.g. with a y- or t-connector 154 for active filtration of particles from the trocar 102 by the filter medium, which in this case can be in the cartridge 150, or in the insufflator 12. The siphon line 136 can instead be connected to any suitable source of suction.

With reference now to Fig. 7, a kit 162 includes a trocar 102 as described above, and a cap 112 as described above, wherein the trocar 102 and cap 112 are attached or separate from one another in the kit. It is also contemplated that the kit can include the tube set 134, and if applicable the external filter unit 138 as described above.

A method includes regulating insufflation of a surgical site with a trocar, e.g. trocar 102, introduced into the surgical site. The method includes siphoning fluid that vents out of the surgical site through the trocar into a tube set, e.g. tube set 134, thereby diverting the fluid from reaching a space external of the surgical site.

This can include capturing liquid droplets, solid particulate, and/or gas from the fluid in a filter medium, e.g. filter medium 140, in a flow path, e.g. flow passage 142, of the fluid. The filter medium is in a fluid circuit connecting between the trocar and an insufflator, e.g. insufflator 112, regulating insufflation with the trocar and/or conducting smoke evacuation through the trocar. The filter medium can be within an insufflator regulating insufflation with the trocar and/or conducting smoke evacuation through the trocar, as shown in Fig. 1, or in an external filter housing in the tube set, as shown in Fig. 6. The method can include evacuating smoke from the surgical site through the trocar and/or regulating stable pneumoperitoneum using the trocar. The trocar 102 can be a first access port, and the method can include accessing the surgical site through second access port, e.g. access port 114 shown in Figs. 1 and 6. Accessing the surgical site through the second access port can include accessing the surgical site without accessing the surgical site through the first access port, i.e. optionally a surgical instrument such as surgical instrument 10, 11 of Figs. 1 and 6 can access the surgical site through the trocar 102 and cap 112, or through a separate access port 14, or through both. The second access port 14 can optionally be connected to an insufflation system.

The methods and systems of the present disclosure, as described above and shown in the drawings, provide for active filtration to prevent particles from within a pneumoperitoneum from entering the operating room air without impeding the performance or effectiveness of the insufflation or stable pneumoperitoneum. While the apparatus and methods of the subject disclosure have been shown and described with reference to preferred embodiments, those skilled in the art will readily appreciate that changes and/or modifications may be made thereto without departing from the scope of the subject disclosure.

## Claims

1. A cap (112) for a trocar (102) assembly comprising:
a housing (114) configured to be removably attached to a proximal portion (108) of a trocar (102), wherein the housing (114) defines an access port opening (118) therethrough configured to be aligned for passage of a surgical instrument through the access port opening (118) and into a main access port (120) of the trocar (102), wherein the housing (114) defines a siphon opening (126) for siphoning particles from the proximal portion (108) of the trocar (102) for filtration, and wherein a plenum (128) is defined in the cap (112) radially outward from the access port opening (118) with a plenum wall (130) separating between the plenum (128) and the access port opening (118), wherein the siphon opening (126) is in fluid communication with the plenum (128) to siphon particles out of the plenum (128), and wherein at least one port (132) is defined through the plenum wall (130) for fluid communication from the access port opening (118) into the plenum (128), wherein the at least one port (132), the plenum (128) and the siphon opening (126) are arranged in a common plane that is perpendicular to a longitudinal axis of the access port opening (118).

2. The cap (112) as recited in claim 1, further comprising a tube set (134) including a siphon tube (136) connected to the siphon opening (126).

3. The cap (112) as recited in claim 2, wherein the siphon tube (136) connects for fluid communication between the housing (114) and an external filter unit (138), wherein the external filter unit (138) houses a filter medium (140) spanning a flow passage (142) through the external filter unit (138) for filtration of flow through the flow passage (142).

4. The cap (112) as recited in claim 3, wherein the external filter unit (138) includes an outlet tube (144) for connecting to an insufflator (12) for active filtration.

5. The cap (112) as recited in claim 2, wherein the tube set (134) includes an insufflation line (146) and a smoke evacuation line (148), each connecting between a cartridge (150) and a multiport connector, wherein the cartridge (150) is configured to connect the tube set (134) to an insufflator, wherein the multiport connector is configured to connect the insufflation line (146) and the smoke evacuation line (148)to a trocar (102) for insufflation and smoke evacuation, and wherein the siphon tube (136) connects to the smoke evacuation line (148)for active filtration of particles from the trocar (102) by a filter medium (140).

6. The cap (112) as recited in claim 5, wherein the cartridge (150) includes an ultra-low particulate air (ULPA) filter medium (140) in fluid communication with the at least one siphon line (136) for filtration of particles from the trocar (102).

7. The cap (112) as recited in claim 1, wherein the housing (114) defines an inlet opening (156) in fluid communication with the access port opening (118), wherein the inlet opening (156) is configured to engage the trocar (102).

8. The cap (112) as recited in claim 7, further comprising a seal (158) extending circumferentially around the inlet opening (156), wherein the seal (158) is configured to engage the proximal portion (108) of the trocar (102) to drive all flow into and out of a main lumen of the trocar (102) through the inlet opening (156).

9. The cap (112) as recited in claim 8, wherein a seal seat (160) is defined about the inlet opening (156) of the housing (114), wherein the seal (158) is seated in the seal seat (160).

10. The cap (112) as recited in claim 1, wherein a distal end of the housing (114) includes at least one inward extending latch member (124) configured to engage a respective rim or detent of the trocar (102) to maintain engagement of the housing(114) to the trocar (102).

11. The cap (112) as recited in claim 10, wherein the at least one inward extending latch member (124) includes a plurality of circumferentially spaced apart, inward extending latch members (124).

12. A trocar assembly (100) comprising:
a trocar (102) including:
an elongated tubular member (104) extending between a distal end (106) configured to be inserted into a surgical site and a proximal portion (108) including a trocar housing (110) configured for introduction of surgical instruments into the tubular member (104), wherein the trocar housing (110) includes at least one latch receptacle (122); and
a cap (112) according to any of the preceding claims.

## Patentansprüche

1. Kappe (112) für eine Trokar-Anordnung (102), umfassend:
- ein Gehäuse (114), das eingerichtet ist, um abnehmbar an einem proximalen Abschnitt (108) eines Trokars (102) befestigt zu werden, wobei das Gehäuse (114) eine durchgehende Zugangsöffnung (118) bildet, die eingerichtet ist, um für einen Durchgang eines chirurgischen Instruments durch die durchgehende Zugangsöffnung (118) und in eine Hauptzugangsöffnung (120) des Trokars (102) ausgerichtet zu werden, wobei das Gehäuse (114) eine Siphonöffnung (126) zum Absaugen von Partikeln aus dem proximalen Abschnitt (108) des Trokars (102) zum Filtern bildet, und wobei in der Kappe (112) radial außerhalb der durchgehenden Zugangsöffnung (118) eine Sammelkammer (128) ausgebildet ist, wobei eine Sammelkammerwand (130) die Sammelkammer (128) und die durchgehende Zugangsöffnung (118) trennt, wobei die Siphonöffnung (126) in Fluidverbindung mit der Sammelkammer (128) steht, um Partikel aus der Sammelkammer (128) abzusaugen, und wobei zumindest eine Öffnung (132) durch die Sammelkammerwand (130) für eine Fluidverbindung von der durchgehenden Zugangsöffnung (118) in die Sammelkammer (128) ausgebildet ist, wobei zumindest eine Öffnung (132), die Sammelkammer (128) und die Siphonöffnung (126) in einer gemeinsamen Ebene angeordnet sind, die senkrecht zu einer Längsachse der durchgehenden Zugangsöffnung (118) verläuft.

2. Kappe (112) nach Anspruch 1, die ferner einen Schlauchsatz (134) mit einem Siphonschlauch (136) aufweist, der mit der Siphonöffnung (126) verbunden ist.

3. Kappe (112) nach Anspruch 2, wobei der Siphonschlauch (136) zur Fluidverbindung das Gehäuse (114) und eine externe Filtereinheit (138) verbindet, wobei die externe Filtereinheit (138) ein Filtermedium (140) aufnimmt, das einen Strömungskanal (142) durch die externe Filtereinheit (138) zum Filtern eines Durchflusses durch den Strömungskanal (142) überspannt.

4. Kappe (112) nach Anspruch 3, wobei die externe Filtereinheit (138) einen Auslassschlauch (144) zum Verbinden mit einem Insufflator (12) zum aktiven Filtern umfasst.

5. Kappe (112) nach Anspruch 2, wobei der Schlauchsatz (134) eine Insufflationsleitung (146) und eine Rauchabsaugleitung (148) umfasst, die jeweils zwischen einer Kartusche (150) und einem Mehrfachanschluss verbunden sind, wobei die Kartusche (150) eingerichtet ist, um den Schlauchsatz (134) mit einem Insufflator zu verbinden, wobei der Mehrfachanschluss eingerichtet ist, um die Insufflationsleitung (146) und die Rauchabsaugleitung (148) mit einem Trokar (102) zur Insufflation und Rauchabsaugung zu verbinden, und wobei der Siphonschlauch (136) mit der Rauchabsaugleitung (148) verbunden ist, um Partikel aus dem Trokar (102) durch ein Filtermedium (140) aktiv zu filtern.

6. Kappe (112) nach Anspruch 5, wobei die Kartusche (150) ein Filtermedium (140) für extrem wenig partikuläre Luft (ULPA) umfasst, das in Fluidverbindung mit der zumindest einen Siphonleitung (136) steht, um Partikel aus dem Trokar (102) zu filtern.

7. Kappe (112) nach Anspruch 1, wobei das Gehäuse (114) eine Einlassöffnung (156) bildet, die in Fluidverbindung mit der durchgehenden Zugangsöffnung (118) steht, wobei die Einlassöffnung (156) eingerichtet ist, um mit dem Trokar (102) in Eingriff zu stehen.

8. Kappe (112) nach Anspruch 7, die ferner eine Dichtung (158) aufweist, die sich in Umfangsrichtung um die Einlassöffnung (156) erstreckt, wobei die Dichtung (158) eingerichtet ist, um mit dem proximalen Abschnitt (108) des Trokars (102) in Eingriff zu stehen, um den gesamten Fluss in ein Hauptlumen des Trokars (102) durch die Einlassöffnung (156) hinein und aus diesem heraus zu leiten.

9. Kappe (112) nach Anspruch 8, wobei eine Dichtungsaufnahme (160) um die Einlassöffnung (156) des Gehäuses (114) herum ausgebildet ist, wobei die Dichtung (158) in der Dichtungsaufnahme (160) aufgenommen ist.

10. Kappe (112) nach Anspruch 1, wobei ein distales Ende des Gehäuses (114) zumindest ein sich nach innen erstreckendes Verriegelungselement (124) umfasst, das eingerichtet ist, um mit einem entsprechenden Rand oder einer Arretierung des Trokars (102) in Eingriff zu stehen, um den Eingriff des Gehäuses (114) mit dem Trokar (102) aufrechtzuerhalten.

11. Kappe (112) nach Anspruch 10, wobei zumindest ein sich nach innen erstreckendes Verriegelungselement (124) eine Mehrzahl von in Umfangsrichtung voneinander beabstandeten, sich nach innen erstreckenden Verriegelungselementen (124) umfasst.

12. Trokar-Anordnung (100), umfassend:
- ein Trokar (102) mit:
- einem länglichen rohrförmigen Element (104), das sich zwischen einem distalen Ende (106), das zum Einführen in eine Operationsstelle eingerichtet ist, und einem proximalen Abschnitt (108) erstreckt, der ein Trokargehäuse (110) umfasst, das zum Einführen von chirurgischen Instrumenten in das rohrförmige Element (104) eingerichtet ist, wobei das Trokargehäuse (110) zumindest eine Verriegelungsaufnahme (122) umfasst; und
- einer Kappe (112) nach einem der vorhergehenden Ansprüche.

## Revendications

1. Capuchon (112) pour un ensemble trocart (102) comprenant :
un boîtier (114) configuré pour être fixé de manière amovible à une partie proximale (108) d'un trocart (102), où le boîtier (114) définit une ouverture d'orifice d'accès (118) à travers celui-ci, configurée pour être alignée pour le passage d'un instrument chirurgical à travers l'ouverture d'orifice d'accès (118) et dans un orifice d'accès principal (120) du trocart (102), où le boîtier (114) définit une ouverture de siphon (126) pour siphonner des particules depuis la partie proximale (108) du trocart (102) pour la filtration, et où un plénum (128) est défini dans le capuchon (112) radialement vers l'extérieur de l'ouverture d'orifice d'accès (118) avec une paroi de plénum (130) séparant le plénum (128) et l'ouverture d'orifice d'accès (118), où l'ouverture de siphon (126) est en communication fluidique avec le plénum (128) pour siphonner des particules hors du plénum (128), et où au moins un orifice (132) est défini à travers la paroi de plénum (130) pour une communication fluidique depuis l'ouverture d'orifice d'accès (118) dans le plénum (128), où l'au moins un orifice (132), le plénum (128) et l'ouverture de siphon (126) sont agencés dans un plan commun qui est perpendiculaire à un axe longitudinal de l'ouverture d'orifice d'accès (118).

2. Capuchon (112) selon la revendication 1, comprenant en outre un ensemble de tubes (134) comprenant un tube de siphon (136) relié à l'ouverture de siphon (126).

3. Capuchon (112) selon la revendication 2, dans lequel le tube de siphon (136) relie le boîtier (114) et une unité de filtration externe (138) pour assurer une communication fluidique entre eux, où l'unité de filtration externe (138) loge un milieu filtrant (140) s'étendant sur un passage d'écoulement (142) à travers l'unité de filtration externe (138) pour la filtration du flux à travers le passage d'écoulement (142).

4. Capuchon (112) selon la revendication 3, dans lequel l'unité de filtration externe (138) comprend un tube de sortie (144) pour être relié à un insufflateur (12) pour une filtration active.

5. Capuchon (112) selon la revendication 2, dans lequel l'ensemble de tubes (134) comprend une ligne d'insufflation (146) et une ligne d'évacuation de fumée (148), chacune se reliant entre une cartouche (150) et un raccord à orifices multiples, où la cartouche (150) est configurée pour relier l'ensemble de tubes (134) à un insufflateur, où le raccord à orifices multiples est configuré pour relier la ligne d'insufflation (146) et la ligne d'évacuation de fumée (148) à un trocart (102) pour l'insufflation et l'évacuation de fumée, et où le tube de siphon (136) se relie à la ligne d'évacuation de fumée (148) pour une filtration active des particules provenant du trocart (102) par un milieu filtrant (140).

6. Capuchon (112) selon la revendication 5, dans lequel la cartouche (150) comprend un milieu filtrant (140) à très faible pénétration des particules (ULPA) en communication fluidique avec l'au moins une ligne de siphon (136) pour la filtration des particules provenant du trocart (102).

7. Capuchon (112) selon la revendication 1, dans lequel le boîtier (114) définit une ouverture d'entrée (156) en communication fluidique avec l'ouverture d'orifice d'accès (118), où l'ouverture d'entrée (156) est configurée pour s'engager avec le trocart (102).

8. Capuchon (112) selon la revendication 7, comprenant en outre un joint d'étanchéité (158) s'étendant de manière circonférentielle autour de l'ouverture d'entrée (156), où le joint d'étanchéité (158) est configuré pour s'engager avec la partie proximale (108) du trocart (102) pour entraîner tout le flux dans et hors d'une lumière principale du trocart (102) à travers l'ouverture d'entrée (156).

9. Capuchon (112) selon la revendication 8, dans lequel un siège de joint d'étanchéité (160) est défini autour de l'ouverture d'entrée (156) du boîtier (114), où le joint d'étanchéité (158) est logé dans le siège de joint d'étanchéité (160).

10. Capuchon (112) selon la revendication 1, dans lequel une extrémité distale du boîtier (114) comprend au moins un élément de verrouillage (124) s'étendant vers l'intérieur, configuré pour s'engager avec un rebord ou un cran respectif du trocart (102) pour maintenir l'engagement du boîtier (114) avec le trocart (102).

11. Capuchon (112) selon la revendication 10, dans lequel l'au moins un élément de verrouillage (124) s'étendant vers l'intérieur comprend une pluralité d'éléments de verrouillage (124) s'étendant vers l'intérieur, espacés de manière circonférentielle.

12. Ensemble trocart (100) comprenant :
un trocart (102) comprenant :
un élément tubulaire allongé (104) s'étendant entre une extrémité distale (106) configurée pour être insérée dans un site chirurgical et une partie proximale (108) comprenant un boîtier de trocart (110) configuré pour l'introduction d'instruments chirurgicaux dans l'élément tubulaire (104), où le boîtier de trocart (110) comprend au moins un réceptacle de verrouillage (122) ; et
un capuchon (112) selon l'une des revendications précédentes.
